# DEMANDE DE BREVET EUROPEEN

(11) **EP 1 743 619 A2**
(43) Date de publication de la demande: **17.01.2007**
(21) Numéro de dépôt: 06300802.3
(22) Date de dépôt: 12.07.2006
(51) Int. Cl.: A61K 8/02, A61K 8/14

(54) **Composition cosmétique à phase lipidique amphiphile**

(30) Priorité: 12.07.2005 FR 0552163
(71) Demandeur: L'ORÉAL, 75008 Paris (FR)
(72) Inventeur: Simonnet, Jean-Thierry, 94240 Cachan (FR)
(74) Mandataire: Le Coupanec, Pascale A.M.P.

(57) **Abrégé**

La présente invention concerne une composition cosmétique de maquillage et/ou de soin comprenant au moins une phase lipidique amphiphile organisée en phase cristal liquide lamellaire ou cubique, caractérisée en ce qu'elle comprend en outre au moins un composé dérivé de l'urée de formule (I) 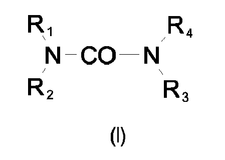 dans laquelle :
R₁, R₂, R₃ et R₄ représentent chacun, indépendamment l'un de l'autre, un atome d'hydrogène, un groupe alkyle en C₁-C₄ ou un groupe hydroxyalkyle en C₂-C₆ pouvant contenir de 1 à 5 groupes hydroxyle, où au moins un des radicaux R₁ à R₄ représente un groupe hydroxyalkyle,
ou un de ses sels, solvates ou isomères,
ledit composé étant présent dans la phase lipidique amphiphile.

## Description

La présente invention a pour objet une composition comprenant au moins une phase lipidique amphiphile organisée en phase cristal liquide lamellaire ou cubique et comprenant en outre au moins un dérivé de l'urée.

On sait que certains lipides amphiphiles possèdent la propriété de former des phases mésomorphes, dont l'état d'organisation est intermédiaire entre l'état cristallin et l'état liquide, selon des phases lamellaires, cubiques ou hexagonales.

En ce qui concerne les phases lamellaires, celles-ci possèdent une structure de type empilement en couche(s) bimoléculaire(s). Lorsqu'une phase lamellaire est dispersée dans un excès d'eau, cette phase lamellaire peut alors former des vésicules. Les vésicules ainsi obtenues se présentent sous la forme de sphérules dont la membrane externe possède une structure lamellaire consistant en une ou plusieurs couches lipidiques, séparées l'une de l'autre par des couches de phase aqueuse. La composition lipidique qui peut être utilisée pour préparer ces sphérules peut être de nature ionique et dans ce cas, on obtient des liposomes ou de nature non ionique et l'on obtient alors des niosomes.

On connaît également des émulsions huile dans eau dont les globules huileux sont enrobés par des lipides amphiphiles organisés selon une phase cristal liquide lamellaire et situés à l'interface huile/phase aqueuse.

Pour sa part, une phase cristal liquide cubique est organisée d'une manière bipolaire en domaines hydrophile et lipophile distincts, en contact étroit et organisés selon un réseau tridimensionnel. Cette phase cristal liquide cubique se présente généralement sous la forme de gel qui peut également être dispersé pour former des particules. Les particules dispersées de phase cristalline liquide cubique possèdent une structure externe lamellaire et une structure interne cubique bi-continue. Cette structure interne possède des domaines hydrophile et lipophile distincts. Ces dispersions de particules sont notamment avantageuses pour la stabilisation de phase huileuse dans des systèmes eau dans huile.

D'une manière générale, les supports à base d'une phase cristal liquide lamellaire, à l'image des liposomes, niosomes ou oléosomes par exemple, ou à base de gel cubique ont particulièrement appréciés pour leurs propriétés d'hydratation et de renfort de la fonction barrière des matières kératiniques et notamment de la peau. Par ailleurs, les phases cristal liquides, en possédant des domaines hydrophiles et hydrophobes, sont particulièrement intéressantes pour solubiliser ou disperser une grande variété de composés hydrophiles, lipophiles et amphiphiles.

Dans tous les cas, les lipides amphiphiles constituant une phase cristal liquide doivent être gonflés (ou hydratés) pour qu'ils puissent former une phase cristal liquide lamellaire ou cubique. Habituellement, cela est fait avec de l'eau ou des glycols (glycérol, propylène glycol, butylène glycol, dipropylène glycol...). Cependant, le potentiel hydratant d'une phase lamellaire hydratée avec de l'eau est insuffisant. L'ajout de glycol dans la phase aqueuse d'hydratation voire l'hydratation des lipides par une phase glycolique pure améliore significativement ces propriétés d'hydratation. Néanmoins, cela s'accompagne d'un effet collant indéniable que l'homme du métier cherche à réduire au maximum.

Il subsiste donc le besoin d'hydrater des lipides amphiphiles, aptes à former une phase cristal liquide lamellaire ou cubique, avec un dérivé au moins aussi efficace que les glycols et beaucoup moins collant, pour profiter des propriétés d'hydratation de telles structures.

Il a maintenant été trouvé que l'addition d'un dérivé d'urée particulier permettait d'obtenir une hydratation appropriée des lipides amphiphiles organisés en phase cristal liquide lamellaire ou cubique en s'affranchissant des inconvénients mentionnés précédemment notamment en terme d'effet collant associés à la présence de glycols tels que le glycérol.

La présente invention a donc pour premier objet une composition cosmétique de maquillage et/ou de soin, notamment de matière(s) kératinique(s), comprenant au moins une phase lipidique amphiphile organisée en phase cristal liquide lamellaire ou cubique, caractérisée en ce qu'elle comprend en outre au moins un composé dérivé de l'urée de formule (I) 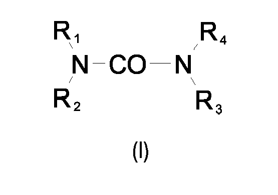 dans laquelle :
R₁, R₂, R₃ et R₄ représentent chacun, indépendamment l'un de l'autre, un atome d'hydrogène, un groupe alkyle en C₁-C₄ ou un groupe hydroxyalkyle en C₂-C₆ pouvant contenir de 1 à 5 groupes hydroxyle, où au moins un des radicaux R₁ à R₄ représente un groupe hydroxyalkyle,
ou un de ses sels, solvates ou isomères,
ledit composé étant présent dans la phase lipidique amphiphile.

Dans une première variante, la composition selon l'invention comprend une phase lipidique amphiphile organisée selon une phase cristal liquide lamellaire dite "phase A" gonflée par au moins un dérivé d'urée de formule (I) telle que définie précédemment.

Selon un premier mode de réalisation de cette variante, ladite composition comprend une dispersion aqueuse de vésicules lipidiques comprenant ladite phase A et en particulier une dispersion aqueuse de liposomes ou de niosomes.

Selon un deuxième mode de réalisation, ladite composition se présente sous la forme d'une émulsion huile dans eau comprenant des vésicules à coeur huileux pourvues d'un enrobage constituée de ladite phase A.

Selon un troisième mode de réalisation, la composition est essentiellement anhydre et comprend au moins une phase grasse notamment liquide et distincte de la phase cristal liquide lamellaire dans laquelle est dispersée ladite phase A.

Au sens de la présente invention, une composition « essentiellement anhydre » signifie une composition selon l'invention contient moins de 7 % en poids d'eau, en particulier moins de 5 % en poids, plus particulièrement moins de 1 % en poids, voire moins de 0,5 % en poids d'eau.

Dans une seconde variante, la composition comprend une dispersion aqueuse de particules d'un gel cubique comprenant au moins un dérivé d'urée de formule (I) telle que définie précédemment.

Selon un mode de réalisation particulier de cette variante, cette composition se présente sous la forme d'une dispersion huile dans eau stabilisée par ladite dispersion de particules.

La présente invention a en outre pour objet, selon un autre de ses aspects, un procédé de traitement cosmétique des matières kératiniques en particulier de la peau, notamment de maquillage et/ou de soin non thérapeutique comprenant au moins l'application d'une composition cosmétique telle que définie précédemment sur les matières kératiniques.

### DERIVES DE L'UREE

Le composé d'urée présent dans la composition selon l'invention est un composé de formule (I) suivante : 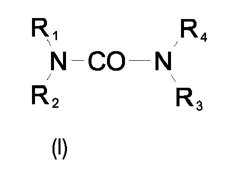 dans laquelle :
R₁, R₂, R₃ et R₄ représentent chacun, indépendamment l'un de l'autre, un atome d'hydrogène, un groupe alkyle en C₁-C₄ ou un groupe hydroxyalkyle en C₂-C₆ pouvant contenir de 1 à 5 groupes hydroxyle et où au moins un des radicaux R₁ à R₄ représente un groupe hydroxyalkyle,
ainsi que leurs sels, leurs solvates, et leurs isomères.

Pour les composés de formule (I) :
- De préférence R₁ désigne un groupe hydroxyalkyle en C₂-C₆ et R₂, R₃, R₄ désignent, indépendamment l'un de l'autre, un atome d'hydrogène ou un groupe alkyle en C₁-C₄ ;
- Préférentiellement R₁ désigne un groupe hydroxyalkyle en C₂-C₆ comprenant de 1 à 5 groupes hydroxyle, notamment 1 groupe hydroxyle, et R₂, R₃, R₄ désignent un atome d'hydrogène ;
- Plus préférentiellement, R₁ désigne un groupe hydroxyalkyle en C₂-C₄ comprenant 1 groupe hydroxyle, et, en particulier, R₂, R₃, R₄ désignent un atome d'hydrogène.

Parmi les groupes alkyle, on peut citer les groupes méthyle, éthyle, n-propyle, isopropyle, n-butyle, iso-butyle et tert-butyle.

Parmi les groupes hydroxyalkyle, on préfère ceux contenant un seul groupe hydroxyle et en particulier les groupes hydroxyéthyle, hydroxypropyle, hydroxybutyle, hydroxypentyle et hydroxyhexyle.

Parmi les sels, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Par solvat, on entend un mélange stoechiométrique dudit composé de formule (I) avec une ou plusieurs molécules d'eau ou de solvant organique, un tel mélange étant issu de la synthèse du composé de formule (I).

Comme composés de formule (I) préférés, on peut citer le N-(2-hydroxyéthyl)-urée; le N-(2-hydroxypropyl)-urée; le N-(3-hydroxypropyl)-urée; le N-(2,3-dihydroxypropyl)- urée ; le N-(2,3,4,5,6-pentahydroxyhexyl)- urée ; le N-méthyl-N-(1,3,4,5,6-pentahydroxy-2-hexyl)- urée ; le N-méthyl-N'-(1-hydroxy-2-méthyl-2-propyl)-urée ; le N-(1-hydroxy-2-méthyl-2-propyl)- urée ; le N-(1,3-dihydroxy-2-propyl)- urée ; le N-(tris-hydroxyméthyl-méthyl)-urée ; le N-éthyl-N'-(2-hydroxyéthyl)- urée ; le N,N-bis-(2-hydroxyéthyl)- urée ; le N,N'-bis-(2-hydroxyéthyl)- urée ; le N,N-bis-(2-hydroxypropyl)- urée ; le N,N'-Bis-(2-hydroxypropyl)- urée ; le N,N-Bis-(2-hydroxyéthyl)-N'-propyl- urée ; le N,N-Bis-(2-hydroxypropyl)-N'-(2-hydroxyéthyl)- urée ; le N-tert.Butyl-N'-(2-(hydroxyéthyl)-N'-(2-(hydroxypropyl)- urée ; le N-(1,3-dihydroxy-2-propyl)-N'-(2-hydroxyéthyl)- urée ; le N,N-Bis-(2-hydroxyéthyl)-N',N'-dimethyl- urée ; le N,N,N',N'-tetrakis-(2-hydroxyéthyl)- urée ; le N',N'-Bis-(2-hydroxyéthyl)-N',N'-bis-(2-hydroxypropyl)- urée ; et leurs mélanges.

De préférence, le composé de formule (I) est le N-(2-hydroxyéthyl)-urée.

Les composés de formule (I) sont des composés connus et notamment décrits dans la demande DE-A-2703185. Parmi ceux-ci, le N-(2-hydroxyéthyl)-urée est en outre disponible dans le commerce, sous forme de mélange à 50 % en poids dans l'eau, auprès de la société NATIONAL STARCH sous la dénomination commerciale Hydrovance^{®}.

Le composé de formule (I) est présent en une quantité efficace, c'est-à-dire suffisante pour procurer l'effet hydratant recherché au niveau de la composition l'incorporant.

Plus précisément, le composé de formule (I) peut être présent dans les compositions selon l'invention en une teneur allant de 1 % à 50 % en poids, par rapport au poids total de la composition, de préférence allant de 2 % à 25 % en poids, et préférentiellement allant de 2 % à 20 % en poids.

Avantageusement, le composé de formule (I) est véhiculé dans une phase aqueuse préalablement à son mélange avec la phase lipidique amphiphile.

### Phase lipidique amphiphile

Au sens de la présente invention, on entend par phase lipidique amphiphile une phase composée d'un lipide ou mélange de lipides amphiphile(s).

Par "lipide amphiphile", on entend ici toutes molécules ayant une structure bipolaire, c'est-à-dire comportant au moins une partie hydrophobe et au moins une partie hydrophile ayant la propriété de réduire la tension interfaciale entre l'eau et une phase huileuse. Les synonymes de lipide amphiphile sont par exemple : tensioactif, agent de surface, émulsionnant.

En ce qui concerne les lipides amphiphiles, utilisables pour former la phase lipidique organisée selon une phase cristal liquide lamellaire ou cubique, il peut s'agir de tout lipide amphiphile connu pour sa capacité à former une phase lamellaire en présence d'eau. Ces lipides sont, de façon connue, des lipides amphiphiles d'origine naturelle ou synthétique, ioniques ou non-ioniques, comportant, par molécule, une ou plusieurs longue(s) chaîne(s) hydrocarbonée(s) saturée(s) ou insaturée(s), linéaire(s) ou ramifiée(s), ayant de préférence 8 à 30 atomes de carbone, ces chaînes étant par exemple une chaîne oléique, lanolique, tétradécylique, hexadécylique, isostéarylique, laurique ou alcoylphényle, et un ou plusieurs groupement(s) hydrophile(s) pris parmi les groupes hydroxyle, étheroxyde, carboxyle, phosphate et amine.

La phase lipidique amphiphile comprend généralement au moins un lipide amphiphile non ionique et le cas échéant un lipide amphiphile anionique.

Les lipides amphiphiles non ioniques de l'invention convenant tout particulièrement sont choisis parmi :
1) les tensioactifs siliconés,
2) les lipides amphiphiles liquides à température inférieure ou égale à 45 °C, choisis parmi les esters d'au moins un polyol d'au moins un acide gras comportant au moins une chaîne alkyle en C₈-C₂₂, saturée ou non saturée, linéaire ou ramifiée, et notamment insaturée ou ramifiée, le polyol étant choisi dans le groupe formé par le polyéthylèneglycol comportant de 1 à 60 unités d'oxyde d'éthylène, le sorbitan, le glycérol pouvant comporter de 2 à 30 unités d'oxyde d'éthylène, les polyglycérols comportant de 2 à 15 unités de glycérol,
3) les esters d'acide gras et de sucre et les éthers d'alcool gras et de sucre,
4) les tensioactifs solides à une température égale à 45°C, choisis parmi les esters gras de glycérol, les esters gras de sorbitan et les esters gras de sorbitan oxyéthylénés, les éthers gras éthoxylés et les esters gras éthoxylés,
5) les copolymères blocs d'oxyde d'éthylène (A) et d'oxyde de propylène (B), et leurs mélanges.
6) Les phospholipides (naturels ou synthétiques) comme la phosphatidylcholine.

1) A titre illustratif des tensioactifs siliconés utilisables selon l'invention, on peut notamment citer des composés siliconés comportant au moins une chaîne oxyéthylénée -OCH₂CH₂- et/ou oxypropylénée -OCH₂CH₂CH₂-. Comme tensioactifs siliconés pouvant être utilisés selon la présente invention, on peut citer ceux décrits dans les documents US-A-5,364,633 et US-A-5,411,744.

En particulier, le tensioactif siliconé utilisé selon la présente invention peut être un composé de formule (II) : 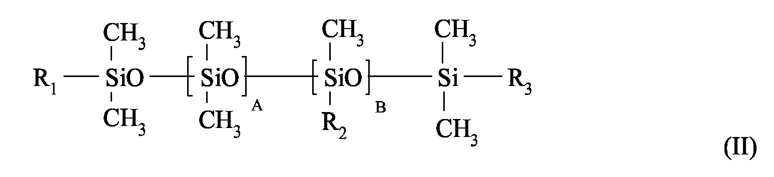 dans laquelle :
R₁, R₂, R₃, indépendamment les uns des autres, représentent un radical alkyle en C₁-C₆ ou un radical -(CH₂)ₓ-(OCH₂CH₂)_{y}-(OCH₂CH₂CH₂)_{z}-OR₄ avec au moins un radical R₁, R₂ ou R₃ n'étant pas un radical alkyle et R₄ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

Selon un mode de réalisation préféré de l'invention, dans le composé de formule (X), le radical alkyle est un radical méthyle, x est un nombre entier allant de 2 à 6 et y est un nombre entier allant de 4 à 30.

On peut également citer, à titre d'exemple de tensioactifs siliconés de formule (II), les composés de formule (III) : 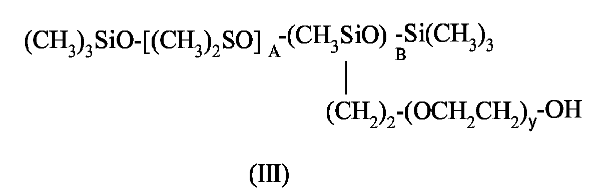 dans laquelle A est un nombre entier allant de 20 à 105, B est un nombre entier allant de 2 à 10 et y est un nombre entier allant de 10 à 20.

On peut également citer à titre d'exemple de tensioactifs siliconés de formule (II), les composés de formule (IV) :

H-(OCH₂CH₂)_{y}-(CH₂)₃-[(CH₃)₂SiO]_{A}'-(CH₂)₃-(OCH₂CH₂)_{y}-OH (IV)

dans laquelle A' et y sont des nombres entiers allant de 10 à 20.

On peut utiliser notamment comme tensioactifs siliconés, ceux commercialisés par la société Dow Corning sous les dénominations DC 5329, DC 7439-146, DC 2-5695 et Q4-3667. Les composés DC 5329, DC 7439-146, DC 2-5695 sont des composés de formule (III) où respectivement A est 22, B est 2 et y est 12 ; A est 103, B est 10 et y est 12 ; A est 27, B est 3 et y est 12.
2) Les lipides amphiphiles liquides à température inférieure ou égale à 45°C peuvent être notamment choisis parmi :
   - l'isostéarate de polyéthylèneglycol de poids molaire 400 (nom CTFA : PEG-8 Isostearate), vendu sous la dénomination Prisorine 3644 par la société UNICHEMA,
   - l'isostéarate de diglycéryle, notamment celui vendu par la société SOLVAY;
   - le laurate de polyglycérol comportant 2 unités de glycérol (polyglyceryl-2 laurate), notamment celui vendu sous la dénomination Diglycerin-monolaurate par la société SOLVAY ;
   - l'oléate de sorbitan, notamment celui vendu sous la dénomination SPAN 80 par la société ICI ;
   - l'isostéarate de sorbitan, notamment celui vendu sous la dénomination NIKKOL SI 10R par la société NIKKO ;
   - le cocoate d'α-butylglucoside ou le caprate d'α-butylglucoside notamment commercialisés par la société ULICE.
3) Les esters d'acide gras et de sucre, utilisables comme lipides amphiphiles non ioniques selon l'invention sont de préférence solides à une température inférieure ou égale à 45°C et peuvent être choisis notamment dans le groupe comprenant les esters ou les mélanges d'esters d'acide gras en C₈-C₂₂ et de sucrose, de maltose, de glucose ou de fructose, et les esters ou les mélanges d'esters d'acide gras en C₁₄-C₂₂ et de méthylglucose. Les acides gras en C₈-C₂₂ ou en C₁₄-C₂₂ formant le motif gras des esters utilisables comportent une chaîne alkyle linéaire saturée ou non saturée, ayant respectivement de 8 à 22 ou de 14 à 22 atomes de carbone. Le motif gras des esters peut être notamment choisi parmi les stéarates, béhénates, arachidonates, palmitates, myristates, laurates, caprates et leurs mélanges. On utilise de préférence des stéarates.

On peut citer, à titre d'exemple d'esters ou de mélanges d'esters d'acide gras et de sucrose, de maltose, de glucose ou de fructose, le monostéarate de sucrose, le distéarate de sucrose, le tristéarate de sucrose et leurs mélanges, tels que les produits commercialisés par la société CRODA sous la dénomination Crodesta F50, F70, F110, F160 ayant respectivement un HLB (Hydrophilic Lipophilic Balance) de 5, 7, 11 et 16 ; et à titre d'exemple d'esters ou de mélanges d'esters d'acide gras et de méthylglucose, le distéarate de méthyl glucose et de polyglycérol-3, vendu par la société GOLDSCHMIDT sous la dénomination de Tego-care 450. On peut citer aussi les monoesters de glucose ou de maltose tels que l'o-hexadécanoyle-6-D-glucoside de méthyle et l'o-hexadécanoyle-6-D-maltoside.

Les éthers d'alcool gras et de sucre, utilisables comme lipides amphiphiles non ioniques sont solides à une température inférieure ou égale à 45 °C et peuvent être choisis notamment dans le groupe comprenant les éthers ou mélanges d'éthers d'alcool gras en C₈-C₂₂ et de glucose, de maltose, de sucrose ou de fructose et les éthers ou mélanges d'éthers d'alcool gras en C₁₄-C₂₂ et de méthylglucose. Ce sont notamment des alkylpolyglucosides.

Les alcools gras en C₈-C₂₂ ou en C₁₄-C₂₂ formant le motif gras des éthers utilisables selon l'invention comportent une chaîne alkyle linéaire saturée ou non saturée, ayant respectivement de 8 à 22 ou de 14 à 22 atomes de carbone. Le motif gras des éthers peut être notamment choisi parmi les motifs décyle, cétyle, béhényle, arachidyle, stéaryle, palmityle, myristyle, lauryle, capryle, hexadécanoyle, et leurs mélanges tels que cétéaryle.

A titre d'exemple d'éthers d'alcool gras et de sucre, on peut citer les alkylpolyglucosides tels que le décylglucoside et le laurylglucoside commercialisés par exemple par la société HENKEL sous les dénominations respectives de Plantaren 2000 et Plantaren 1200, le cétostéarylglucoside éventuellement en mélange avec l'alcool cétostéarylique, commercialisé par exemple sous la dénomination Montanov 68 par la société SEPPIC, sous la dénomination Tego-care CG90 par la société GOLDSCHMIDT et sous la dénomination Emulgade KE3302 par la société HENKEL, ainsi que l'arachidylglucoside, par exemple sous la forme du mélange d'alcools arachidique et béhénique et d'arachidylglucoside, commercialisé sous la dénomination Montanov 202 par la société SEPPIC.

On utilise plus particulièrement comme lipide amphiphile non ionique de ce type, le monostéarate de sucrose, le distéarate de sucrose, le tristéarate de sucrose et leurs mélanges, le distéarate de méthylglucose et de polyglycérol-3 et les alkylpolyglucosides.
4) Les esters gras de glycérol, utilisables comme lipides amphiphiles non ioniques selon l'invention, solides à une température égale à 45 °C, peuvent être choisis notamment dans le groupe comprenant les esters formés d'au moins un acide comportant une chaîne alkyle linéaire saturée, ayant de 16 à 22 atomes de carbone, et de 1 à 10 motifs glycérol.

Ces esters peuvent être notamment choisis parmi les stéarates, béhénates, arachidates, palmitates et leurs mélanges. On utilise de préférence des stéarates et palmitates.

On peut citer à titre d'exemple de ce type de tensioactifs, les monostéarate, distéarate, tristéarate et pentastéarate de décaglycérol (10 unités de glycérol) (noms CTFA : Polyglyceryl-10 stéarate, Polyglyceryl-10 distéarate, Polyglyceryl-10 tristéarate, Polyglyceryl-10 pentastéarate) tels que les produits vendus sous les dénominations respectives Nikkol Decaglyn 1-S, 2-S, 3-S et 5-S par la société NIKKO, et le monostéarate de diglycérol (nom CTFA : Polyglyceryl-2 stearate) tel que le produit vendu par la société NIKKO sous la dénomination NIKKOL DGMS.

Les esters gras de sorbitan, utilisables comme lipides amphiphiles non ioniques selon l'invention, solides à une température égale à 45 °C, sont choisis notamment dans le groupe comprenant les esters d'acide gras en C₁₆-C₂₂ et de sorbitan et les esters d'acide gras en C₁₆-C₂₂ et de sorbitan oxyéthylénés. Ils sont formés d'au moins un acide gras comportant au moins une chaîne alkyle linéaire saturée, ayant respectivement de 16 à 22 atomes de carbone, et de sorbitol ou de sorbitol éthoxylé. Les esters oxyéthylénés comportent généralement de 1 à 100 unités d'oxyde d'éthylène et de préférence de 2 à 40 unités d'oxyde d'éthylène (OE).

Ces esters peuvent être notamment choisis parmi les stéarates, béhénates, arachidates, palmitates, et leurs mélanges. On utilise de préférence des stéarates et palmitates.

On peut citer à titre d'exemple d'ester gras de sorbitan et d'ester gras de sorbitan oxyéthyléné, utilisable selon l'invention, le monostéarate de sorbitan (nom CTFA : Sorbitan stearate) vendu par la société ICI sous la dénomination Span 60, le monopalmitate de sorbitan (nom CTFA : Sorbitan palmitate) vendu par la société ICI sous la dénomination Span 40, le tristéarate de sorbitan 20 OE (nom CTFA : Polysorbate 65) vendu par la société ICI sous la dénomination Tween 65.

Les éthers gras éthoxylés solides à une température égale à 45°C, utilisables comme lipides amphiphiles non ioniques utilisables selon l'invention sont de préférence des éthers formés de 1 à 100 unités d'oxyde d'éthylène et d'au moins une chaîne d'alcool gras ayant de 16 à 22 atomes de carbone. La chaîne grasse des éthers peut être notamment choisie parmi les motifs béhényle, arachidyle, stéaryle, cétyle, et leurs mélanges tels que cétéaryle. A titre d'exemple d'éthers gras éthoxylés, on peut citer les éthers d'alcool béhénique comprenant 5, 10, 20 et 30 unités d'oxyde d'éthylène (noms CTFA : Beheneth-5, Beheneth-10, Beheneth-20, Beheneth-30), tels que les produits commercialisés sous les dénominations Nikkol BB5, BB10, BB20, BB30 par la société NIKKO, et l'éther d'alcool stéarylique comprenant 2 unités d'oxyde d'éthylène (nom CTFA : Steareth-2), tel que le produit commercialisé sous la dénomination Brij 72 par la société ICI.

Les esters gras éthoxylés solides à une température égale à 45 °C, utilisables comme lipides amphiphiles non ioniques selon l'invention sont des esters formés de 1 à 100 unités d'oxyde d'éthylène et d'au moins une chaîne d'acide gras comportant de 16 à 22 atomes de carbone. La chaîne grasse des esters peut être notamment choisie parmi les motifs stéarate, béhénate, arachidate, palmitate, et leurs mélanges. A titre d'exemple d'esters gras éthoxylés, on peut citer l'ester d'acide stéarique comprenant 40 unités d'oxyde d'éthylène, tel que le produit commercialisé sous la dénomination Myrj 52 (nom CTFA : PEG-40 stearate) par la société ICI ainsi que l'ester d'acide béhénique comprenant 8 unités d'oxyde d'éthylène (nom CTFA : PEG-8 behenate), tel que le produit commercialisé sous la dénomination Compritol HD5 ATO par la société GATTEFOSSE.
5) Les copolymères blocs d'oxyde d'éthylène et d'oxyde de propylène, utilisables comme lipides amphiphiles non ioniques selon l'invention peuvent être choisis notamment parmi les copolymères blocs de formule (V) :

   HO(C₂H₄O)ₓ(C₄H₆O)_{y}(C₂H₄O)_{z}H (V)

   dans laquelle x, y et z sont des nombres entiers tels que x+z va de 2 à 100 et y va de 14 à 60, et leurs mélanges, et plus particulièrement parmi les copolymères blocs de formule (V) ayant un HLB allant de 2 à 16.

Ces copolymères blocs peuvent être notamment choisis parmi les poloxamers et notamment parmi le Poloxamer 231 tel que le produit commercialisé par la société ICI sous la dénomination Pluronic L81 de formule (V) avec x=z=6, y=39 (HLB 2) ; le Poloxamer 282 tel que le produit commercialisé par la société ICI sous la dénomination Pluronic L92 de formule (V) avec x=z=10, y=47 (HLB 6) ; et le Poloxamer 124 tel que le produit commercialisé par la société ICI sous la dénomination Pluronic L44 de formule (V) avec x=z=11, y=21 (HLB 16).

Comme lipides amphiphiles non ioniques, on peut aussi citer les mélanges de tensioactifs non ioniques décrits dans le document EP-A-705593 incorporé ici pour référence.

Parmi les lipides amphiphiles non ioniques, on peut utiliser en particulier :
- le PEG-8 Isostéarate (comportant 8 moles d'oxyde d'éthylène),
- l'hexadécyl éther de triglycéryle,
- le distéarate de sucrose,
- l'isostéarate de diglycéryle liquide,
- le monolaurate de polyglycérol comportant 2 unités de glycérol et les stéarates de polyglycérol comportant de 2 à 10 unités de glycérol, et en particulier ceux comportant 3 unités de glycérol,
- l'oléate de sorbitan liquide,
- le palmitate de sorbitan,
- le stéarate de sorbitan comportant 4 moles d'oxyde d'éthylène,
et leurs mélanges.

Les lipides amphiphiles non ionique peuvent être présents dans les compositions selon l'invention en une teneur allant de 0,2 % à 15 % en poids, par rapport au poids total de la composition, et de préférence allant de 0,2 % à 10 % en poids, et préférentiellement allant de 0,2 % à 8 % en poids.

Conviennent tout particulièrement à titre de lipides amphiphiles non ioniques, les tensioactifs solides à une température inférieure ou égale à 45°C, choisis parmi les esters gras de glycérol, les esters gras de sorbitan et les esters gras de sorbitan oxyéthylénés, les éthers gras éthoxylés et les esters gras éthoxylés.

Selon un mode de réalisation particulier de l'invention, les compositions de l'invention peuvent contenir en outre un ou plusieurs lipides amphiphiles ioniques, en particulier un ou plusieurs lipides anioniques ou cationiques, différents des lipides amphiphiles non ioniques décrits précédemment.

Ainsi, les lipides amphiphiles anioniques pouvant être utilisés dans l'invention sont choisis de préférence parmi :
- les sels alcalins du dicétyl- et du dimyristyl-phosphate ;
- les sels alcalins du cholestérol sulfate ;
- les sels alcalins du cholestérol phosphate ;
- les lipoaminoacides et leurs sels tels que les acylglutamates mono- et di-sodiques comme le sel disodique de l'acide N-stéaroyl L-glutamique commercialisé sous la dénomination Acylglutamate HS21 par la société AJINOMOTO ;
- les sels de sodium de l'acide phosphatidique ;
- les phospholipides ;
- les dérivés alkylsulfoniques notamment de formule : 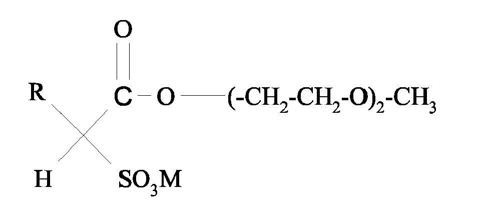
dans laquelle R représente des radicaux alkyle en C₁₆-C₂₂, en particulier les radicaux C₁₆H₃₃ et C₁₈H₃₇ pris en mélange ou séparément et M est un métal alcalin ou alcalino-terreux tel que le sodium ;
et leurs mélanges.
Les lipoaminoacides et leurs sels, et notamment ceux cités précédemment, s'avèrent particulièrement avantageux dans le cadre de l'invention.

Les lipides amphiphiles cationiques pouvant être utilisés selon l'invention sont choisis, de préférence, dans le groupe formé par les sels d'ammonium quaternaire, les amines grasses et leurs sels.

En ce qui concerne les sels d'ammonium quaternaires, on peut plus particulièrement citer les sels de formule générale suivante : 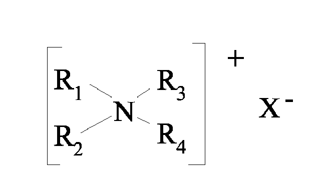 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone; X est un anion choisi dans le groupe des halogénures, phosphates, acétates, lactates, alkyl(C₂-C₆)sulfates, alkyl-ou-alkylarylsulfonates,

Le chlorure de béhényltriméthylammonium convient tout particulièrement à l'invention à titre de sel d'ammonium quaternaire.

Une composition selon l'invention peut comprendre de 0,01 % à 6 % en poids, et de préférence de 0,2 % à 4 % en poids de lipide(s) amphiphile(s) ionique(s), par rapport au poids total de la composition.

Plus généralement, la teneur totale de lipides amphiphiles non ioniques et ioniques va de 0,25 % à 18 % en poids, par rapport au poids total de la composition, et de préférence va de 1 % à 10 % en poids.

### Phase cristal liquide lamellaire

Comme précisé précédemment, selon une première variante, une composition conforme à l'invention comprend une phase lipidique organisée selon une phase cristal liquide lamellaire.

Par "phase cristal liquide lamellaire" on entend, dans le cadre de la présente invention, un empilement de bicouches lipidiques gonflées.

Une description de référence est donnée dans "The Colloïdal Domain" second edition, de D.Fennell Evans et H.Wennerström , édité par Wiley-VCH (1999), pages 295-296 et 306-307.
a) Selon une première variante, ce type de composition est une dispersion aqueuse de vésicules lipidiques comprenant une phase cristal liquide lamellaire.

On entend par « vésicules ou (sphérules) lipidiques comprenant une phase liquide lamellaire », des particules formées d'une membrane constituée par un ou plusieurs feuillets concentriques, ces feuillets comportant une ou plusieurs couches bimoléculaires de lipides amphiphiles formant la phase cristal liquide lamellaire encapsulant une phase aqueuse. La phase aqueuse peut contenir des substances actives hydrosolubles et les couches bimoléculaires de lipides amphiphiles peuvent contenir des substances actives lipophiles.

Ces vésicules ont généralement un diamètre moyen compris entre 10 et 5000 nanomètres. De telles dispersions aqueuses de vésicules lipidiques, encore dénommées liposomes ou niosomes sont notamment décrites dans les documents FR 2 532 191, FR 2 694 893, FR 2 714 596 et FR 2 730 931 dont les contenus sont incorporés par référence.

Les vésicules à coeur aqueux selon l'invention, de préférence, comprennent une membrane lipidique formée à partir d'au moins un lipide amphiphile non ionique et d'au moins un lipide amphiphile ionique gonflés par un dérivé d'urée de formule (I) telle que décrite précédemment.

Les dispersions de vésicules à coeur aqueux peuvent être préparées par tout procédé connu de fabrication des vésicules lipidiques amphiphiles et plus particulièrement par le procédé dit "par cofusion des lipides" ce qui permet de les préparer de façon simple à l'échelle industrielle.
b) Selon un deuxième mode de réalisation de cette variante, les compositions selon l'invention comprennent une émulsion huile dans eau formée de vésicules à coeur huileux pourvus d'un enrobage phase cristal liquide lamellaire, c'est-à-dire contenant des vésicules comprenant une membrane lipidique encapsulant une phase huileuse.

Les vésicules à coeur huileux se présentent de préférence sous la forme de globules huileux en dispersion unitairement enrobés d'une couche monolamellaire ou oligolamellaire (2 à 10 lamelles lipidiques) obtenue à partir d'au moins un lipide amphiphile non ionique ayant une HLB comprise entre 2 et 5 et d'au moins un lipide amphiphile non ionique ayant une HLB comprise entre 8 et 12.

Des exemples d'agents tensioactifs ayant un HLB allant de 2 à 5 sont le distéarate de sucrose, le distéarate de diglycéryle, le tristéarate de tétraglycéryle, le décastéarate de décaglycéryle, le monostéarate de diglycérol, le tristéarate d'hexaglycéryle, le pentastéarate de décaglycéryle, le monostéarate de sorbitane, le tristéarate de sorbitane, le monostéarate de diéthylène glycol, l'ester de glycérol et d'acides palmitique et stéarique, le monostéarate polyoxyéthylèné 2 OE (comportant 2 motifs oxyéthylène), le mono et dibéhénate de glycéryle, le tétrastéarate de pentaérythrytol.

On peut citer comme exemples de tensioactifs ayant un HLB allant de 8 à 12 les composés suivants : le monostéarate de sorbitane polyoxyéthyléné 4 OE, le tristéarate de sorbitane polyoxyéthyléné 20 OE, le monostéarate polyoxyéthyléné 8 OE, le monostéarate d'hexaglycéryle, le monostéarate polyoxyéthyléné 10 OE et le distéarate polyoxyéthylèné 12 OE, le distéarate de méthylglucose polyoxyéthyléné 20 OE.

Les vésicules lipidiques à coeur huileux conformes à l'invention peuvent être préparées selon le procédé de fabrication décrit dans les demandes FR 2709666 et FR 2725369. Ce procédé consiste dans une première étape à mélanger sous agitation la phase grasse contenant le lipide amphiphile non ionique de HLB 2-5, le lipide amphiphile de HLB 8-12 dans une deuxième étape à soumettre le mélange obtenu à une homogénéisation basée sur le principe de la cavitation. Cette homogénéisation est obtenue soit à l'aide de hautes pressions comprises entre 200 et 1500 bars, soit à l'aide d'ultra-sons, soit à l'aide d'homogénéisateurs équipés d'une tête rotor-stator.

La taille moyenne des globules huileux revêtus est inférieure à 1 000 nanomètres, et de préférence inférieure ou égale à 800 nanomètres.

De telles émulsions sont notamment décrites dans les documents FR2709666, FR2725369 et FR2767691 dont les contenus sont incorporés par référence.
c) Selon un troisième mode de réalisation, les compositions selon l'invention sont essentiellement anhydres et comprennent au moins une phase grasse et au moins une phase cristal liquide lamellaire gonflée par au moins un dérivé d'urée de formule (I) décrite précédemment.

Ainsi, il est connu que certains mélanges de lipides amphiphiles connus pour former, par contact avec une phase aqueuse, des vésicules constituées de feuillets plus ou moins sphériques de phase lipidique vésiculaire encapsulant la phase aqueuse, conservent cette propriété de former des vésicules, lorsqu'ils sont sous forme de phase lamellaire et sont mélangés à une phase grasse, généralement utilisée pour la fabrication de compositions cosmétiques essentiellement anhydres, qui contient des huiles organiques et/ou minérales, des corps gras, le plus souvent des cires, et des tensioactifs.

De telles compositions sont notamment décrites dans le document EP 534 823 dont le contenu est incorporé par référence.

Les vésicules discutées précédemment sont par ailleurs particulièrement avantageuses à titre de véhicules.

Ainsi, les vésicules des compositions selon l'invention peuvent contenir, de façon connue, un ou plusieurs composé(s) actif(s) ayant une activité cosmétique et/ou dermopharmaceutique, qui, selon leurs caractéristiques de solubilité, peuvent avoir différentes localisations. Si les actifs sont hydrosolubles, on les introduit, de préférence, dans la phase aqueuse encapsulée des vésicules à coeur aqueux. Si les actifs sont liposolubles, on les introduit, de préférence, dans la phase lipidique constituant la membrane ou bien dans la phase huileuse encapsulée des vésicules à coeur huileux ou bien dans la phase cristal liquide lamellaire présente dans la composition essentiellement anhydre.

Si les actifs sont amphiphiles, ils se répartissent entre la phase lipidique et la phase aqueuse encapsulée des vésicules à coeur aqueux ou bien entre la phase lipidique et la phase aqueuse de la dispersion des vésicules à coeur huileux, avec un coefficient de partage qui varie selon la nature de l'actif amphiphile et les compositions respectives des différentes phases en contact avec l'actif

Les actifs hydrosolubles sont, par exemple, la glycérine, le sorbitol, l'érythrulose et les antibiotiques. Les actifs liposolubles ou partiellement liposolubles (amphiphiles) sont choisis parmi ceux qui n'augmentent pas de façon significative la perméabilité des vésicules, ne provoquent pas leur floculation et leur fusion et ne diminuent pas le taux d'encapsulation. On utilise avantageusement des actifs liposolubles qui constituent également des additifs.

Les actifs liposolubles préférés selon l'invention sont choisis dans le groupe formé par :
- les sphingomyélines,
- les glycocéramides, en particulier ceux issus de germe de blé et
- les céramides naturels ou de synthèse, de préférence ceux décrits dans la demande de brevet français n° 2673179.

Par ailleurs, on peut, de façon connue, incorporer dans la phase lipidique constituant la membrane lipidique des vésicules à coeur aqueux, au moins un additif qui a pour fonction principale de diminuer la perméabilité des vésicules, de prévenir leur floculation et leur fusion et d'augmenter le taux d'encapsulation.

On peut ainsi ajouter à la phase lipidique au moins un additif choisi, parmi notamment les stérols et en particulier les phytostérols et le cholestérol.

### Phase cristal liquide cubique

Selon la seconde variante de l'invention, la composition comprend une phase lipidique amphiphile organisée sous la forme d'une phase cristal liquide cubique.

Une phase cubique est organisée d'une manière bipolaire en domaines hydrophile et lipophile distincts, en contact étroit et formant un réseau tridimensionnel thermodynamiquement stable. Une telle organisation a été notamment décrite dans "La Recherche", Vol. 23, PP. 306-315, Mars 1992 et dans "Lipid Technology", Vol. 2, n°2, pp. 42-45, Avril 1990. Selon l'arrangement des domaines hydrophile et lipophile, la phase cubique est dite de type normal ou inverse. Le terme phase cubique utilisé selon la présente invention regroupe bien entendu les différents types de phases cubiques.

D'une façon générale, les phases Cristal Liquides sont décrites dans "The aqueous phase behavior of surfactants" de R.G.Laughlin édité chez Academic Press (1994), dans le chapitre 8.4 p 200-237.

Selon un premier mode de réalisation, cette composition comprend une dispersion aqueuse et stable de particules de gels cubiques notamment présentant simultanément des domaines hydrophiles et lipophiles.

Généralement, les particules à phase cristal liquide cubiques sont formées par fragmentation et dispersion d'un gel à phase cristal liquide cubique. Ces phases cristal liquides cubiques peuvent être obtenues à partir des lipides amphiphiles tels que définis précédemment sous réserve que ceux-ci s'avèrent efficaces pour conduire à ce type d'architecture. Ainsi, certains lipides amphiphiles sont capables de former à l'état pur des phases cubiques, alors que d'autres nécessitent la présence d'un solvant (eau ou solvant organique) ou d'un composé organique. De tels gels peuvent notamment être préparés selon le protocole décrit dans le document WO 02/02716 dont le contenu est incorporé par référence.

La fragmentation du gel est effectuée avec un agent de fragmentation.

L'agent de fragmentation peut être un hydrotrope c'est-à-dire un composé apte à interrompre les phases cristal liquide, comme décrit dans Pearson, J.T., Smith, J.M., "The effects of hydrotropic salts on the stability of liquid crystalline systems", J. Pharm. Pharmac., 26, 123(124 (1974). Les hydrotropes peuvent être choisis parmi les alcools, les polyols, les alcools éthoxylés, les copolymères d'oxyde d'éthylène et d'oxyde de propylène, et les acides gras éthoxylés. Comme composés hydrotropes, on peut en particulier citer l'éthanol, le 1,4-butanediol, le 1,2-hexanediol, l'éthylène glycol, le propylène glycol, et le glycérol. De tels composés hydrotropes sont notamment décrits dans le document WO-A-02/02716.

L'agent de fragmentation peut également être choisi parmi les polymères amphiphiles tels que les copolymères blocs tribloc de polyoxyde d'éthylène/polyoxyde de propylène/polyoxyde d'éthylène vendu sous les dénomination PLURONIC par la société BASF ; la polyvinylpyrrolidone ; les protéines amphiphiles comme la caséine, et les glycoprotéines.

L'agent de fragmentation peut également être choisi parmi :
- les tensioactifs non ioniques polyoxyéthylénés comme les alkyléthers oxyéthylénés, les alkylesters oxyéthylénés comme par exemple les alkyls esters de sorbitan polyoxyéthyéléns par au moins 20 moles d'oxyde d'éthylène tels que le palmitate de sorbitan 20 OE vendu sous la dénomination "MONTANOX 40 DF" par la société SEPPIC, le laurate de sorbitan 20 OE vendu sous la dénomination "TWEEN 20"par la société SEPPIC, le PEG-20 stéarate, le laureth-23, l'oleth-20 ; les alkyl esters de polyglycérol, oxyéthylénés ou non, tels que le polyglycéryle-10 laurate vendu sous la dénomination "Decaglyn 1-L" par la société Nikko Chemicals.
- les tensioactifs anioniques comme les sulfates d'alkyl éthers tels que le lauryléther sulfate de sodium ; les sulfates d'alkyl ester tel que les esters de l'acide iséthionique ainsi que ses sels et notamment le cocoyl iséthionate de sodium vendu sous la dénomination "GEROPON AC 78" par la société RHODIA ;
- les tensioactifs cationiques comme les alkyltriméthylammoniums , de préférence ceux ayant un groupe alkyle en C₁₂-C₂₂, comme par exemple le chlorure de cocoyltriméthylammonium et le bromure de cétyltrriméthylammonium ;
- les tensioactifs amphotères comme les alkyl bétaïnes telles que la lauryl amidopropyl bétaïne et l'oléyl amidopropyl bétaïne.

De tels agents de fragmentation sont notamment décrits dans les demandes WO-A-93/06921, FR-A-2720937.

A titre de dispersion, aqueuse et stable, de particules de gel cubique, on peut plus particulièrement citer celles à base de 3,7,11,15-tétraméthyl 1,2,3-hexadecanetriol ou phytantriol. Le phytantriol est un composé connu qui est notamment commercialisé sous la dénomination de "Phytantriol-63926" par la Société Roche. Une telle dispersion est notamment décrite dans le document FR 2 720 937 dont le contenu est incorporé par référence.

Selon un autre mode de réalisation, la composition selon l'invention se présente sous la forme d'une dispersion d'une phase huileuse dans une phase aqueuse stabilisée par une telle dispersion cubique.

Une telle composition est notamment décrite dans le document FR 2 726 762 dont le contenu est incorporé par référence.

### Phase grasse

D'une manière générale, les compositions selon l'invention comprennent en outre une phase grasse et notamment une phase grasse liquide.

Ainsi, les compositions cosmétiques de l'invention comportent généralement au moins une phase grasse liquide et notamment au moins un corps gras liquide à température ambiante (25 °C) et à pression atmosphérique et/ou un corps gras solide à température ambiante et à pression atmosphérique. La phase grasse peut en outre contenir des agents gélifiants et structurants d'huiles de nature organique et/ou des solvants organiques lipophiles.

La phase grasse du produit selon l'invention peut notamment comprendre, à titre de corps gras liquide, au moins une huile volatile ou non volatile ou un de leurs mélanges.

Par "huile volatile", on entend au sens de l'invention toute huile susceptible de s'évaporer au contact de la peau ou des lèvres en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,01 à 300 mm de Hg (1,33 Pa à 40.000 Pa) et de préférence supérieure à 0,3 mm de Hg (30 Pa).

Par "huile non volatile", on entend une huile restant sur la peau ou les lèvres à température ambiante et pression atmosphérique au moins plusieurs heures et ayant notamment une pression de vapeur inférieure à 0,01 mm de Hg (1,33 Pa).

Ces huiles volatiles ou non volatiles peuvent être des huiles hydrocarbonées notamment d'origine animales ou végétales, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permethyls^{®}, les esters ramifiés en C₈-C₁₆ tels que le néopentanoate d'iso-hexyle, et leurs mélanges. D'autres huiles hydrocarbonées volatiles comme les distillats de pétrole, notamment ceux vendus sous la dénomination Shell Solt® par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 7 atomes de silicium, ces silicones comportant éventuellement des groupes alkyle ou alcoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment les huiles de germe de blé, de tournesol, de pépins de raisin, de sésame, de maïs, d'abricot, d'amande d'abricot, de ricin, de karité, d'avocat, d'olive, de soja, d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société STEARINERIES DUBOIS ou ceux vendus sous les dénominations Miglyol 810^{®}, 812^{®} et 818^{®} par la société DYNAMIT NOBEL,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane et leurs mélanges,
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans le produit conforme à l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates et leurs mélanges.

La phase grasse liquide et notamment l'huile ou mélange d'huiles peut représenter de 0,01 % à 99 % du poids total de la composition, en particulier de 0,05 % à 60 % et plus particulièrement de 2 % à 40 % en poids par rapport au poids total de la composition.

La composition peut comprendre au moins une phase grasse solide et en particulier au moins une cire. Cette phase grasse solide ou la cire peut représenter de 0,01 à 70 %, notamment de 0,1 à 65 % et en particulier de 1 à 50 % en poids, par rapport au poids total de la phase grasse.

Les cires utilisables dans l'invention sont des composés solides à température ambiante, destinés à structurer la composition en particulier sous forme de stick ; elles peuvent être hydrocarbonées, fluorées et/ou siliconées et être d'origine végétale, minérale, animale et/ou synthétique. En particulier, elles présentent une température de fusion supérieure à 40 °C et mieux supérieure à 45 °C.

Comme cire utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 40 °C et mieux à 45 °C, les cires de silicones comme les alkyl- ou alkoxy-diméthicones ayant une chaîne alkyle ou alcoxy de 10 à 45 atomes de carbone, les esters de poly(di)méthylsiloxane solide à 40 °C dont la chaîne ester comporte au moins 10 atomes de carbone ; et leurs mélanges.

Les compositions conformes à l'invention et notamment de type dispersion aqueuse contiennent généralement de l'eau et/ou au moins un solvant hydrosoluble.

Par "solvant hydrosoluble", on désigne un composé liquide à température ambiante et miscible à l'eau (miscibilité dans l'eau supérieure à 50 % en poids à 25 °C et pression atmosphérique).

Les solvants hydrosolubles considérés sont généralement en outre volatils. Parmi ces solvants hydrosolubles, on peut citer notamment les mono-alcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3 butylène glycol et le di-propylène glycol, les cétones en C₃ et C₄ et les aldéhydes en C₂ à C₄.

Les compositions de l'invention, peuvent comprendre en outre au moins un agent de coloration pouvant notamment être présent à raison de 0,01 % à 40 % en poids, notamment de 0,01 % à 30 % en poids et en particulier de 0,05 % à 25 % en poids, par rapport au poids total du produit.

Ces ou cet agent de coloration peuvent être choisis parmi les pigments, les colorants hydrosolubles ou liposolubles, les nacres, les paillettes et leurs mélanges.

Par pigments, il faut comprendre des particules blanches ou colorées, minérales ou organiques, insolubles dans la phase hydrophile liquide, destinées à colorer et/ou opacifier la composition. Par nacres, il faut comprendre des particules irisées, notamment produites par certains mollusques dans leur coquille ou bien synthétisées.

On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi le mica recouvert de titane ou d'oxychlorure de bismuth, le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth. On peut également utiliser les pigments interférentiels, notamment à cristaux liquides ou multicouches.

Les colorants hydrosolubles sont par exemple le jus de betterave, le bleu de méthylène.

Les compositions selon l'invention peuvent, de plus, comprendre tous les ingrédients classiquement utilisés dans les domaines concernés et plus spécialement dans le domaine cosmétique et dermatologique. Ces ingrédients sont en particulier choisis parmi les vitamines, les antioxydants, les épaississants, les oligo-éléments, les adoucissants, les séquestrants, les parfums, les agents alcalinisants ou acidifiants, les conservateurs, les filtres UV, les actifs hydrophiles ou lipophiles et leurs mélanges. Les quantités de ces différents ingrédients sont celles classiquement utilisées dans les domaines concernés et par exemple de 0,01 % à 20 % du poids total de la composition.

De façon connue, la composition selon l'invention peut également contenir des agents actifs habituels dans le domaine cosmétique ou dermatologique. On peut citer en particulier tous les actifs connus pour leur activité sur le vieillissement de la peau comme les agents keratolytiques ou prodesquamants, par exemple les α-hydroxy-acides, les β-hydroxy-acides, les α-ceto-acides, les rétinoïdes et leurs esters, le rétinol, l'acide rétinoïque et ses dérivés On peut citer aussi les vitamines, telles que par exemple les vitamines C, B3 ou PP, B5, E et les dérivés de ces vitamines et notamment leurs esters; la vitamine K et ses dérivés (K1, K2,...); les agents anti-radicaux libres; les filtres solaires; les agents hydratants comme les polyols; les céramides; la DHEA et ses dérivés; le coenzyme Q10; les agents blanchissants et dépigmentants comme l'acide kojique, les dérivés de para-aminophénols, l'arbutine et leurs dérivés, et leurs mélanges.

Les compositions selon l'invention peuvent se présenter sous forme d'une émulsion ou dispersion, notamment d'émulsions ou dispersions huile-dans-eau (H/E). Ces composition sont préparées selon les méthodes usuelles. De préférence, la composition est une dispersion huile-dans-eau.

Selon une variante de l'invention, la composition est essentiellement anhydre. Elle peut alors se présenter sous une forme coulée de forme stick par exemple dans le cas d'un rouge à lèvres.

En outre, les compositions utilisées selon l'invention peuvent être plus ou moins fluides et avoir l'aspect d'une crème blanche ou colorée, d'une pommade, d'un lait, d'une lotion, d'un sérum.

La composition de l'invention peut être par exemple utilisée pour le soin, le traitement, le maquillage des matières kératiniques (notamment d'être humain), en particulier de la peau (notamment du visage et/ou du cuir chevelu), des lèvres, des cheveux.

Ainsi, la composition selon l'invention peut être un produit pour le soin de la peau, notamment pour le visage, le cou, le contour de l'oeil, le corps.

La composition de maquillage selon l'invention peut être un produit de maquillage des lèvres (rouge à lèvres), un fond de teint, un fard à joues, un fard à paupières, un eye-liner, un produit anti-cernes, un produit de maquillage du corps.

Avantageusement, la composition est une composition non rincée.

La composition de l'invention peut aussi être utilisée pour le soin et/ou le traitement des cheveux.

L'invention a donc aussi pour objet l'utilisation cosmétique d'une composition telle que définie ci-dessus pour le soin et/ou le traitement des cheveux.

La composition selon l'invention permet notamment une bonne hydratation de la peau, des muqueuses et/ou du cuir chevelu, et est particulièrement adaptée au traitement de la peau sèche.

Un autre objet de l'invention est donc un procédé cosmétique de soin et/ou d'hydratation et/ou de maquillage de la peau, des lèvres, caractérisé en ce qu'on applique sur la peau, les lèvres une composition telle que définie ci-dessus.

Les exemples qui suivent sont donnés à titre illustratif, mais non limitatif, de la présente invention.

### Exemple 1 : Mélange de lipides amphiphiles gonflé avec de l'N-(2-hydroxyéthyl)- urée pour former une phase cristal liquide lamellaire

| | |
|---|---|
| - Hexadécyl ether de triglycéryle | 22,5 % |
| - Cholestérol | 22,5 % |
| Phosphate de dicétyle | 5,0 % |
| - N-(2-hydroxyéthyl)-urée en solution aqueuse à 50 % en poids commercialisé par NATIONAL STARCH sous le nom d'Hydrovance^{®} | 50,0 % |

Après fusion à 110 °C des lipides amphiphiles et obtention d'un mélange homogène, la température est descendue à 90 °C et la N-(2-hydroxyéthyl)-urée est ajoutée progressivement, sous agitation. Après totale incorporation de N-(2-hydroxyéthyl)-urée, on obtient une pâte homogène blanche correspondant à une phase cristal liquide lamellaire. Observée au microscope optique, en polarisation, on obtient une image conforme à ce type de structure (cf "The aqueous phase behavior of surfactants" de R.G.Laughlin édité chez Academic Press (1994) pages 539 et 560).

Ainsi, la N-(2-hydroxyéthyl)-urée se trouve au niveau des têtes polaires des lipides amphiphiles formant les bicouches lipidiques.

### Exemple 2 : Produit de soin à base de liposomes non ioniques hydratés avec de l'N-(2-hydroxyéthyl)- urée

### Phase 1

| | |
|---|---|
| - Palmitate de sorbitan (SPAN 40^{®}-Uniqema) | 3,8 % |
| - Cholesterol | 3,8 % |
| - Sel monosodique de l'acide N-Stéaroyl L Glutamique (Acyglutamate HS 11^{®}- Ajinomoto) | 0,4 % |

### Phase 2

| | |
|---|---|
| - N-(2-hydroxyéthyl)-urée en solution aqueuse à 50 % en poids commercialisé par NATIONAL STARCH sous le nom d'Hydrovance^{®} | 10,0 % |

### Phase 3

| | |
|---|---|
| - Eau déminéralisée | 45,0 % |
| - Conservateurs | 0,3 % |

### Phase 4

| | |
|---|---|
| - Huile d'amande d'abricot | 5,0 % |
| - Stéarate d'isocétyle | 8,0 % |
| - Cyclométhicone (D5) | 10,0 % |

### Phase 5

| | |
|---|---|
| - Hostacerin AMPS® (Clariant) | 1,2 % |
| - Eau distillée | 12,5 % |

Les phases 1 et 2 sont mélangées, comme dans l'exemple 1. Ce mélange est ensuite amené à 45° C puis dispersé sous très vive agitation (rotor ―stator) dans la phase 3. On obtient alors une suspension de liposomes non ioniques, qu'il est ensuite possible d'homogénéiser à l'aide d'un homogénéisateur haute pression ( Niro-Soavi OBL20) à une pression variant de 20.10⁶ et 60.10⁶ Pascal. On disperse ensuite la phase 4 sous rotor stator dans la dispersion 1+2+3 et pour optimiser la dispersion, on homogénéise entre 20.10⁶ et 60.10⁶ Pascal. On obtient alors une suspension d'huile stabilisée par des vésicules non ioniques hydratées par de l'hydroxy ethyl urée. Après introduction de la phase 5, on obtient une émulsion de soin hydratant, efficace et moins collante que la même composition contenant du glycérol à la place de l'hydroxy ethyl urée.

### Exemple 3 : Emulsion lamellaire de type Oléosomes (globules huileux enrobés par une phase cristal liquide lamellaire)

### Phase A1 :

| | |
|---|---|
| - Distéarate de sucrose (commercialisé par la Société "STEARINERIE DUBOIS") | 2,0 % |
| - Stéarate de sorbitane oxyéthyléné à 4 moles d'oxyde d'éthylène (commercialisé sous le nom de "TWEEN 61" par la Société ICI) | 1,4 % |
| - Acide stéarique | 0,75 % |
| - heptanoate de Stearyle (commercialisé par la société DRAGOCO sous le nom de PCL solide®) | 5,50 % |
| - Vaseline codex | 2,10 % |
| - Huile d'avocat | 4,50 % |
| - Huile de jojoba | 4,10 % |
| - Huile de silicone volatile | 3,70 % |
| - Acétate de Vitamine E | 0,50 % |
| - D α-tocophérol (commercialisé par la Société HENKEL sous le nom de "COPHEROL 1300®") | 0,30 % |

### Phase A2 :

| | |
|---|---|
| - Gomme de silicone (commercialisée par la Société DOW CORNING sous le nom "Q2-1403 Fluid") | 4,00 % |
| - Parfum | 0,3 % |
| - Propylparaben | 0,1 % |

### Phase B :

| | | |
|---|---|---|
| - N-(2-hydroxyéthyl)-urée en solution aqueuse à 50 % en poids (commercialisé par NATIONAL STARCH sous le nom Hydrovance®) | | 10,00 % |
| - Méthyl paraben | | 0,30 % |
| - Triéthanolamine | | 0,40 % |
| - Eau déminéralisée | q.s.p. | 100,00 % |

### Phase C :

| | |
|---|---|
| - Mélange de polymères carboxyvinyliques (commercialisé sous le nom de "CARBOPOL 980" par la Société GOODRICH) | 0,30 % |
| - Eau déminéralisée | 9,70 % |

Les deux phases A1 et B sont portées à 65 °C avant d'être associées (B dans A1) sous très vive agitation (rotor-stator). Après contrôle d'une parfaite dispersion, il est possible, optionnellement, d'homogénéiser la dispersion entre 20.10⁶ Pascal et 60.10⁶ Pascal, pour obtenir une dispersion dont la taille des globules d'huile est inférieure à 500 nm. La phase cristal liquide lamellaire les enrobant ayant été hydratée avec une solution aqueuse d'hydroxy ethyl urée. On disperse à température ambiante la phase A2 dans la première dispersion, sous vive agitation. La phase C, préalablement préparée, est ensuite dispersée afin de gélifier la suspension.

On obtient une émulsion hydratante adaptée pour les peaux sèches et moins collante que la même émulsion contenant du glycérol en lieu et place d'Hydrovance.

### Exemple 4 : Stick de rouge à lèvres contenant une phase cristal liquide lamellaire hydratée avec de l'N-(2-hydroxyéthyl)- urée

On introduit, dans une composition de rouge à lèvres telle que ci-dessous décrite, 10 % de la préparation donnée dans l'exemple 1.

### Base de rouge à lèvres :

| | |
|---|---|
| - Polybutylène | 6,00 % |
| - Huile de lanoline | 20,00 % |
| - Octoxyglycerylbehenate | 20,00 % |
| - Stéaryl heptanoate | 9.80 % |
| - Huile de ricin | 17,00 % |
| - Huile de Jojoba | 9,80 % |
| - Antioxydant | 0,15 % |
| - Pigments | 7,25 % |
| - Phase cristal liquide lamellaire de l'exemple 1 | 10,00 % |

Une fois la base de rouge à lèvres préparée, à 100-110 °C, la préparation de l'exemple 1 est introduite à 80 °C, sous agitation type défloculeuse, jusqu'à obtention d'une base colorée et homogène. Les sticks sont ensuite coulés. Lors de l'application sur les lèvres, des liposomes non ioniques gonflés avec la N-(2-hydroxyéthyl)-urée sont formés, permettant ainsi d'hydrater les lèvres.

### Exemple 5 : Crème de soin comprenant une dispersion de particules de gel cubique.

On prépare une émulsion comprenant une dispersion de particules de gel cubique ayant la composition suivante :

| | | |
|---|---|---|
| Phytantriol | | 3 g |
| Hydroxyéthylurée en solution aqeuse à 50 % en poids (Hydrovance de la société National Starch) | | 1,2 g |
| Conservateur | | 0,9 g |
| Eau distillée | qsp | 100 g |
| Huile de jojoba | | 7,5 g |
| Huile de vaseline | | 2,5 g |
| Cyclopentasiloxane | | 10g |

La solution aqueuse d'hydroxyéthyl urée et le phytantriol sont mélangés pour obtenir un gel transparent compact de phase cubique. Celui-ci est ensuite dispersé dans la phase aqueuse sous vive agitation au rotor-stator. Un passage en homogénéisateur haute pression à 500 bars, est réalisé pour améliorer l'homogénéité de la dispersion. La fraction huileuse est ensuite ajoutée et dispersée au rotor-stator. On effectue à nouveau 1 passage du mélange dans l'homogénéisateur haute pression à 500 bars. On obtient une suspension fluide, sprayable et stable, dont la taille des gouttes d'huile est de l'ordre de 400 nm.

## Revendications

1. Composition cosmétique de maquillage et/ou de soin comprenant au moins une phase lipidique amphiphile organisée en phase cristal liquide lamellaire ou cubique, **caractérisée en ce qu'**elle comprend en outre au moins un composé dérivé de l'urée de formule (I) 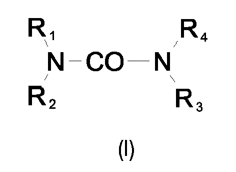 dans laquelle :
R₁, R₂, R₃ et R₄ représentent chacun, indépendamment l'un de l'autre, un atome d'hydrogène, un groupe alkyle en C₁-C₄ ou un groupe hydroxyalkyle en C₂-C₆ pouvant contenir de 1 à 5 groupes hydroxyle, où au moins un des radicaux R₁ à R₄ représente un groupe hydroxyalkyle,
ou un de ses sels, solvates ou isomères,
ledit composé étant présent dans la phase lipidique amphiphile.

2. Composition selon la revendication 1, **caractérisée en ce que** R₁ désigne un groupe hydroxyalkyle en C₂-C₆ et R₂, R₃, R₄ désignent, indépendamment l'un de l'autre, un atome d'hydrogène ou un groupe alkyle en C₁-C₄;

3. Composition selon la revendication 1 ou 2, **caractérisée en ce que** R₁ désigne un groupe hydroxyalkyle en C₂-C₆ comprenant de 1 à 5 groupes hydroxyle, et R₂, R₃, R₄ désignent un atome d'hydrogène.

4. Composition selon l'une quelconque des revendications précédentes, **caractérisée en ce que** R₁ désigne un groupe hydroxyalkyle en C₂-C₆ comprenant 1 groupe hydroxyle.

5. Composition selon l'une quelconque des revendications précédentes, **caractérisée en ce que** R₁ désigne un groupe hydroxyalkyle en C₂-C₄ comprenant 1 groupe hydroxyle et R₂, R₃, R₄ désignent un atome d'hydrogène.

6. Composition selon l'une quelconque des revendications précédentes, **caractérisée par le fait que** le composé de formule (I) est choisi parmi le N-(2-hydroxyéthyl)-urée ; le N-(2-hydroxypropyl)-urée ; le N-(3-hydroxypropyl)-urée ; le N-(2,3-dihydroxypropyl)- urée ; le N-(2,3,4,5,6-pentahydroxyhexyl)- urée ; le N-méthyl-N-(1,3,4,5,6-pentahydroxy-2-hexyl)- urée ; le N-méthyl-N'-(1-hydroxy-2-méthyl-2-propyl)-urée ; le N-(1-hydroxy-2-méthyl-2-propyl)- urée ; le N-(1,3-dihydroxy-2-propyl)- urée ; le N-(tris-hydroxyméthyl-méthyl)-urée ; le N-éthyl-N'-(2-hydroxyéthyl)- urée ; le N,N-bis-(2-hydroxyéthyl)- urée; le N,N'-bis-(2-hydroxyéthyl)- urée ; le N,N-bis-(2-hydroxypropyl)- urée ; le N,N'-Bis-(2-hydroxypropyl)- urée ; le N,N-Bis-(2-hydroxyéthyl)-N'-propyl- urée ; le N,N-Bis-(2-hydroxypropyl)-N'-(2-hydroxyéthyl)-urée; le N-tert.Butyl-N'-(2-(hydroxyéthyl)-N'-(2-(hydroxypropyl)- urée; le N-(1,3-dihydroxy-2-propyl)-N'-(2-hydroxyéthyl)- urée ; le N,N-Bis-(2-hydroxyéthyl)-N',N'-dimethyl- urée ; le N,N,N',N'-tetrakis-(2-hydroxyéthyl)- urée; le N',N'-Bis-(2-hydroxyéthyl)-N',N'-bis-(2-hydroxypropyl)- urée ; et leurs mélanges.

7. Composition selon l'une quelconque des revendications précédentes, **caractérisée par le fait que** le composé de formule (I) est le N-(2-hydroxyéthyl)-urée.

8. Composition selon l'une quelconque des revendications précédentes, **caractérisée par le fait que** les sels des composés de formule (I) sont choisis parmi les sels de l'acide sulfurique, l'acide chlorhydrique, l'acide bromhydrique, l'acide iodhydrique, l'acide phosphorique, l'acide borique, l'acide propionique, l'acide acétique, l'acide téréphtalique, l'acide citrique, l'acide tartrique.

9. Composition selon l'une quelconque des revendications précédentes, **caractérisée par le fait que** le composé de formule (I) est présent en une teneur allant de 1 % à 50 % en poids, en particulier allant de 2 % à 25 % en poids, et préférentiellement allant de 2 % à 20 % en poids par rapport au poids total de la composition.

10. Composition selon l'une quelconque des revendications précédentes, **caractérisée en ce que** la phase lipidique amphiphile comprend au moins un lipide amphiphile non ionique et le cas échéant un lipide amphiphile anionique.

11. Composition selon la revendication précédente, **caractérisée en ce que** le lipide amphiphile non ionique est choisi parmi :
- les tensioactifs siliconés,
- les lipides amphiphiles liquides à température égale à 45 °C, choisis parmi les esters d'au moins un polyol d'au moins un acide gras comportant au moins une chaîne alkyle en C₈-C₂₂, saturée ou non saturée, linéaire ou ramifiée, le polyol étant choisi dans le groupe formé par le polyéthylèneglycol comportant de 1 à 60 unités d'oxyde d'éthylène, le sorbitan, le glycérol pouvant comporter de 2 à 30 unités d'oxyde d'éthylène, les polyglycérols comportant de 2 à 15 unités de glycérol,
- les esters d'acide gras et de sucre et les éthers d'alcool gras et de sucre,
- les tensioactifs solides à une température égale à 45 °C, choisis parmi les esters gras de glycérol, les esters gras de sorbitan et les esters gras de sorbitan oxyéthylénés, les éthers gras éthoxylés et les esters gras éthoxylés,
- les copolymères blocs d'oxyde d'éthylène (A) et d'oxyde de propylène (B), et
- les phospholipides
- leurs mélanges.

12. Composition selon l'une quelconque des revendications précédentes, **caractérisée en ce qu'**elle comprend au moins de 0,2 % à 15 % en poids, et de préférence de 0,2 % à 10 % en poids de lipide(s) amphiphile(s) non ionique(s), par rapport au poids total de la composition.

13. Composition selon l'une quelconque des revendications précédentes, **caractérisée en ce qu'**elle comprend au moins un lipide amphiphile ionique.

14. Composition selon la revendication précédente, **caractérisée par le fait que** le lipide amphiphile ionique est choisi parmi :
- les sels alcalins du dicétyl- et du dimyristyl-phosphate ;
- les sels alcalins du cholestérol sulfate ;
- les sels alcalins du cholestérol phosphate ;
- les lipoaminoacides et leurs sels ;
- les sels de sodium de l'acide phosphatidique ;
- les phospholipides ;
- les dérivés alkylsulfoniques ;
- les sels d'ammonium quaternaires
- les amines grasses et leurs sels, et
- leurs mélanges.

15. Composition selon l'une quelconque des revendications précédentes, **caractérisée en ce qu'**elle comprend de 0,01 % à 6 % en poids, et de préférence de 0,2 % à 4 % en poids de lipide(s) amphiphile(s) ionique(s), par rapport au poids total de la composition.

16. Composition selon l'une quelconque des revendications précédentes, **caractérisée par le fait que** la teneur totale de lipides amphiphiles non ioniques et ioniques va de 0,25 % à 18 % en poids, par rapport au poids total de la composition, et de préférence va de 1 % à 10 % en poids.

17. Composition selon l'une quelconque des revendications précédentes, **caractérisée en ce qu'**elle comprend une phase lipidique organisée selon une phase cristal liquide lamellaire.

18. Composition selon la revendication précédente, **caractérisée en ce qu'**elle comprend une dispersion aqueuse de vésicules lipidiques comprenant ladite phase cristal lipidique lamellaire.

19. Composition selon la revendication 17, **caractérisée en ce qu'**elle est anhydre et comprend en outre une phase grasse distincte de la phase cristal liquide lamellaire.

20. Composition selon l'une quelconque des revendications 18 à 19, **caractérisée en ce que** les vésicules sont des liposomes ou des niosomes.

21. Composition selon l'une quelconque des revendications 1 à 17, **caractérisée en ce qu'**elle se présente sous la forme d'une émulsion huile dans eau, comprenant des vésicules à coeur huileux pourvus d'un enrobage constitué de ladite phase cristal liquide lamellaire.

22. Composition selon l'une quelconque des revendications 1 à 16, **caractérisée en ce qu'**elle comprend une dispersion aqueuse stable de particules de gel cubique.

23. Composition selon la revendication précédente, **caractérisée en ce qu'**elle se présente sous la forme d'une dispersion huile dans eau stabilisée par ladite dispersion de particules de gel cubique.

24. Composition selon l'une quelconque des revendications précédentes, **caractérisée en ce qu'**elle comprend une phase grasse liquide.

25. Composition selon l'une quelconque des revendications précédentes, **caractérisée en ce qu'**elle comprend au moins un agent de coloration.

26. Procédé de traitement cosmétique de matière(s) kératinique(s), **caractérisé en ce qu'**on applique sur ladite matière kératinique au moins une composition selon l'une quelconque des revendications précédentes.

27. Procédé cosmétique de maquillage de la peau comprenant l'application sur la peau d'une composition selon l'une quelconque des revendications précédentes.
